# EUROPÄISCHE PATENTSCHRIFT

(11) **EP 1 722 995 B1**
(45) Veröffentlichungstag und Bekanntmachung des Hinweises auf die Patenterteilung: **26.09.2007**
(21) Anmeldenummer: 05701040.7
(22) Anmeldetag: 19.01.2005
(51) Int. Cl.: B60K 7/00

(54) **RADANTRIEB**
WHEEL DRIVE
ENTRAINEMENT DE ROUE

(30) Priorität: 11.02.2004 DE 102004006722; 12.05.2004 DE 102004023341
(43) Veröffentlichungstag der Anmeldung: 22.11.2006
(73) Patentinhaber: ZF FRIEDRICHSHAFEN AG, 88038 Friedrichshafen (DE)
(72) Erfinder: SCHARFENBERG, Stephan, 99869 Tüttleben (DE)
(86) Internationale Anmeldenummer: PCT/EP2005/000486
(87) Internationale Veröffentlichungsnummer: WO 2005/077696

(56) Entgegenhaltungen:
- EP-A- 0 507 137
- DE-A1- 10 130 100
- DE-A1- 19 904 552
- DE-A1- 19 949 351

## Beschreibung

Die Erfindung bezieht sich auf einen Radantrieb nach der im Oberbegriff von Anspruch 1 näher definierten Art.

Gattungsgemäße Radantriebe, insbesondere Einrad-Triebwerke für Flurförderfahrzeuge, weisen einen Antriebsmotor, welcher über ein Untersetzungsgetriebe das Antriebsrad antreibt, und einen Lenkmotor auf, mittels welchem das Antriebsrad um eine Lenkachse verdreht werden kann, um eine Lenkbewegung auszuführen. Hierbei ist der Einbauraum für den Radantrieb sowie den Lenkantrieb äußerst begrenzt.

Die DE 34 20 164 A1 offenbart einen Radantrieb für ein Flurförderfahrzeug, bei welchem ein Antriebsrad von einem Antriebsmotor über ein Untersetzungsgetriebe angetrieben wird, und ein Lenkmotor über eine Kette den Radantrieb um eine Lenkdrehachse verdrehen kann, um eine Lenkbewegung auszuführen. Hierbei ist der Lenkmotor separat neben dem Fahrmotor angeordnet, wodurch ein großer Einbauraum benötigt wird.

Ein weiterer Radantrieb ist aus DE19904552 A1 (beinhaltet die Merkmale des Oberbegriffs des Anspruch 1) bekannt.

Der vorliegenden Erfindung liegt die Aufgabe zugrunde, einen Radantrieb, insbesondere für ein Flurförderfahrzeug, zu schaffen, bei welchem der Fahrmotor das Fahrzeugrad antreibt und der Radantrieb über einen Lenkmotor um eine Lenkdrehachse drehbar antreibbar und kompakt und kostengünstig aufgebaut ist.

Die Aufgabe wird mit einem, auch die kennzeichnenden Merkmale des Hauptanspruchs aufweisenden, gattungsgemäßen Radantrieb gelöst.

Erfindungsgemäß sind der Fahrmotor, der Lenkmotor und eine Bremse, welche das Antriebsrad bremst, koaxial angeordnet. Vorzugsweise befindet sich die Bremse zwischen dem Fahrmotor und dem Lenkmotor. Durch die Verwendung eines Lenkgetriebes, welches vorzugsweise ebenfalls koaxial zum Lenkmotor angeordnet ist, ist es möglich, einen kompakten Lenkmotor zu verwenden.

In einer weiteren Ausgestaltung der Erfindung sind der Fahrmotor, die Bremse und der Lenkmotor in einem gemeinsamen Gehäuse angeordnet, wodurch eine weitere Verringerung des benötigten Bauraums möglich ist und eine kostengünstige Lösung erreicht wird.

In einer weiteren Ausgestaltungsform der Erfindung wird die Bremse als sogenannte Negativ-Bremse ausgeführt, wodurch die Bremse über Federkraft im Schließsinne betätigbar ist und durch hydraulischen Druck oder elektrische Betätigung einer Magnetspule im Öffnungssinne betätigt werden kann. Die Bremse kann als Reibscheibenbremse ausgeführt sein, wobei die Reibbeläge entweder in einem Raum, welcher mit Schmiermittel gefüllt ist, angeordnet sein können oder auch als trockenlaufende Lamellenbremse ohne Schmiermittel ausgeführt sein kann.

In einer weiteren Ausgestaltungsform ist die Antriebswelle des Fahrmotors über eine Mitnahmeverzahnung mit einem Bauteil, der sogenannten Bremsennabe, verbunden, welche mit den drehenden Teilen der Bremse verbunden ist. Diese Mitnahmeverzahnung kann auch über eine Paßfeder ausgeführt sein.

In einer weiteren Ausgestaltungsform der Erfindung ist die als Negativ-Bremse ausgeführte Bremse über Schraubendruckfedern oder über eine Tellerfeder im Schließsinne betätigbar.

In einer weiteren Ausgestaltungsform der Erfindung ist die Antriebswelle des Lenkmotors mit einem inneren Zentralrad eines Planetengetriebes verbunden oder mit diesem einstückig ausgeführt, welches mit Planetenrädern in Wirkverbindung steht. Die Planetenräder stehen mit einem ersten Hohlrad und einem zweiten Hohlrad in Wirkverbindung, wobei eines der Hohlräder drehfest mit dem Fahrzeugchassis und das andere Hohlrad mit dem Abtriebsrad in Verbindung stehen. Indem die beiden Hohlräder unterschiedliche Zähnezahlen aufweisen, ist das Planetengetriebe als Wolfrom-Trieb ausgeführt, wodurch sich bei Drehung des inneren Zentralrades das Antriebsrad um seine Lenkdrehachse dreht und somit eine Lenkbewegung ausführt.

Weitere Merkmale sind der Figuren-Beschreibung zu entnehmen.
Es zeigen:

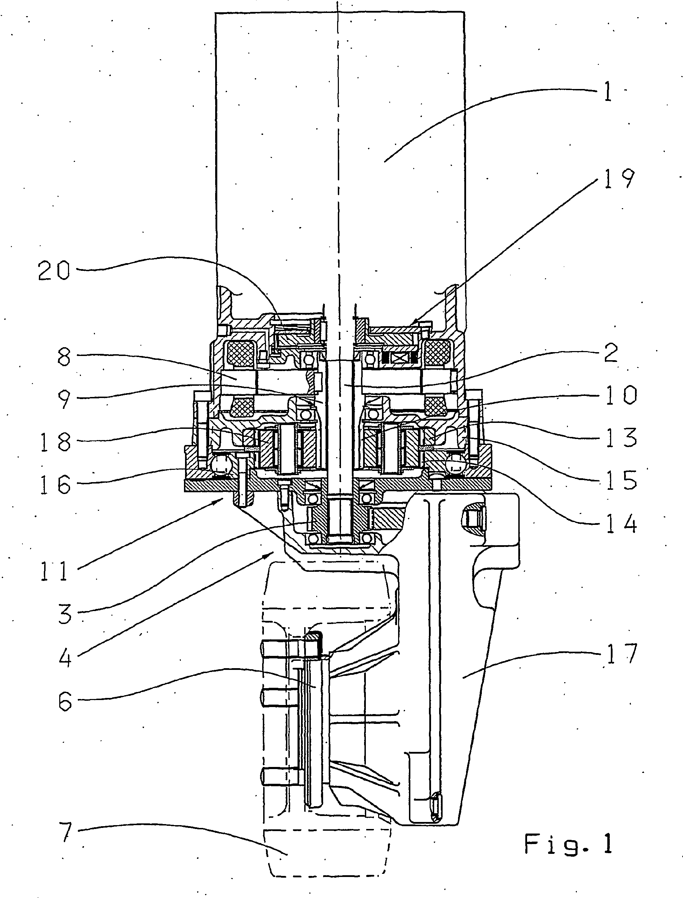

- Fig. 1: eine Ansicht des gesamten Radantriebs;
- Fig. 2: einen Detail-Ausschnitt eines Halbschnittes des Radantriebs, bei welchem die Bremse, der Lenkmotor und das Lenkgetriebe dargestellt sind;
- Fig. 3: einen Teil-Ausschnitt im Halbschnitt des Radantriebs, bei welchem die Bremse, der Lenkmotor und das Lenkgetriebe dargestellt sind;

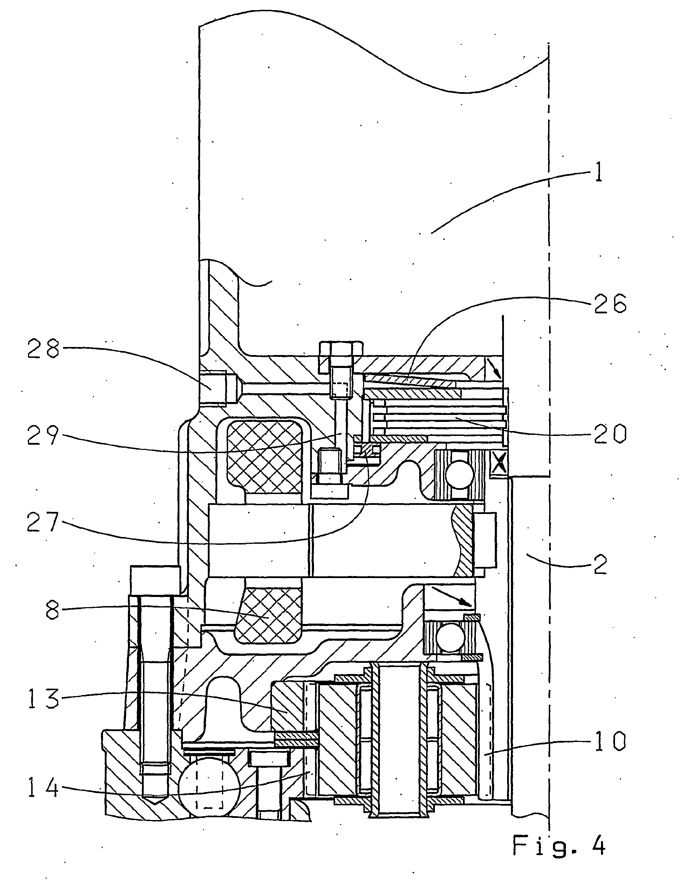

- Fig. 4: einen Ausschnitt im Halbschnitt des Radantriebs, bei welchem die Bremse, der Lenkmotor und das Lenkgetriebe dargestellt sind und
- Fig. 5: einen Ausschnitt im Halbschnitt des Antriebsmotors, bei welchem die Bremse, der Lenkmotor und das Lenkgetriebe dargestellt sind.

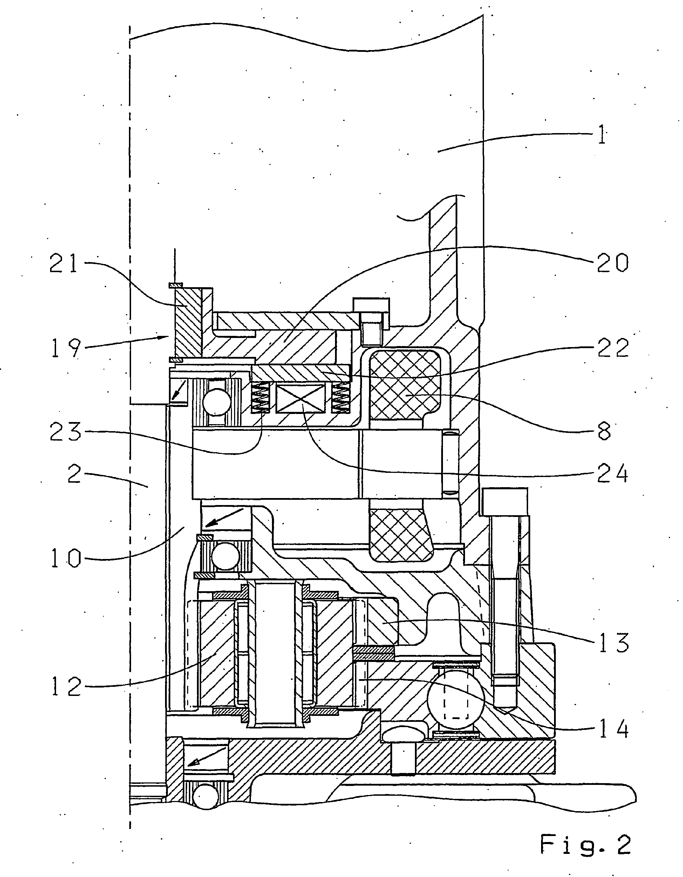

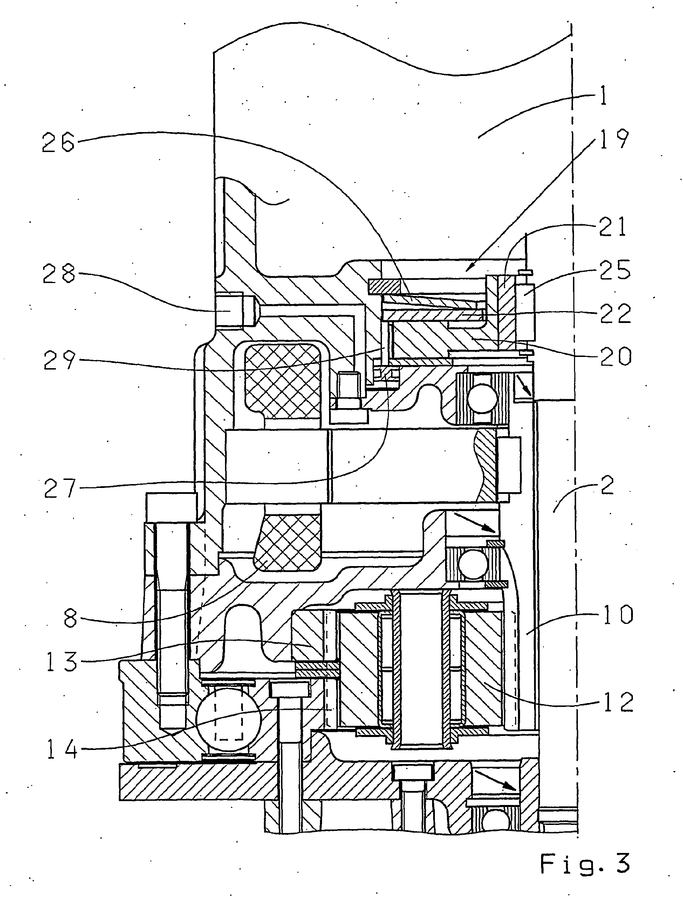

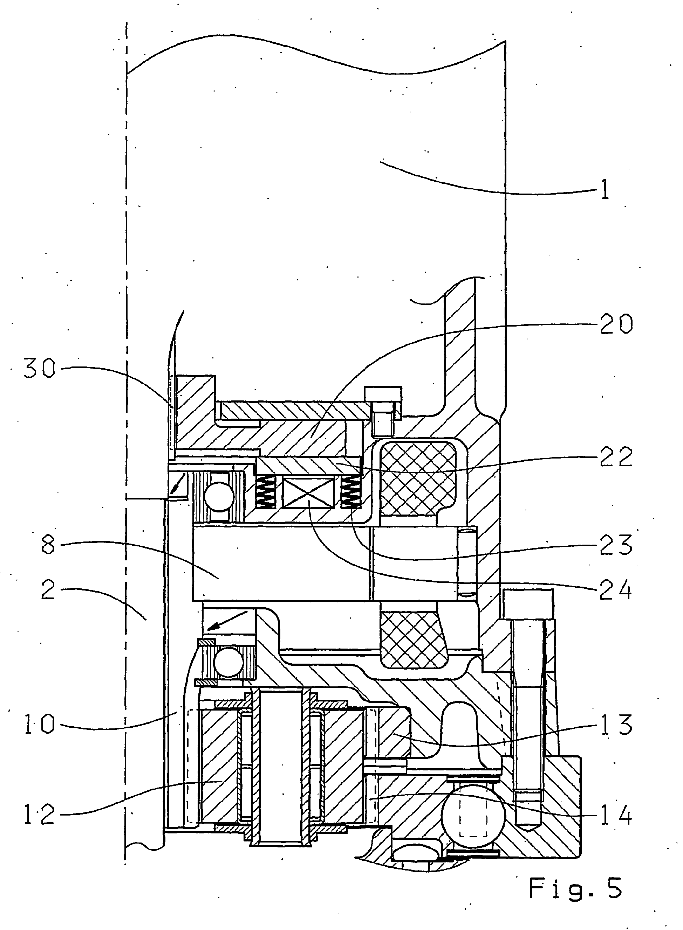

Fig. 1:
   Ein Antriebsmotor 1, welcher vorzugsweise als Elektromotor ausgeführt ist, treibt über eine Antriebswelle 2 ein erstes Stirnrad 3 eines Untersetzungsgetriebes 4 an. Das erste Stirnrad 3 treibt ein zweites Stirnrad 5 an, welches über ein nicht dargestelltes Kegelradgetriebe den Abtrieb 6 des Radantriebs antreibt, welcher mit einem Antriebsrad 7 verbunden ist. Ein zweiter Antriebsmotor 8 treibt über seine Antriebswelle 9 ein inneres Zentralrad 10 eines Planetengetriebes 11 an, welches als Wolfrom-Getriebe ausgeführt ist. Die Planeten 12 kämmen mit einem ersten Hohlrad 13 und einem zweiten Hohlrad 14, wobei das erste Hohlrad 13 drehfest in einem Deckel 15 gehalten ist, welcher drehfest mit einem Bauteil des Fahrzeugchassis in Verbindung steht. Das zweite Hohlrad 14 ist drehfest mit einem Deckel 16 verbunden, welcher drehfest mit dem Gehäuse 17 verbunden ist, wodurch sich bei Drehung des zweiten Hohlrads 14 das Gehäuse 17 im Sinne einer Lenkbewegung dreht. Das Lenkgetriebe 18 ist zwischen dem Untersetzungsgetriebe 4 und dem zweiten Antriebsmotor 8 angeordnet. Eine Bremse 19 ist zwischen dem ersten Antriebsmotor 1 und dem zweiten Antriebsmotor 2 angeordnet. Der erste Antriebsmotor 1, der zweite Antriebsmotor 2, die Bremse 19 und das Lenkgetriebe 18 sind koaxial angeordnet. Die Antriebswelle 2 ist mit den drehenden Teilen 20 der Bremse 19 verbunden.
Fig. 2:
   Die Antriebswelle 2 des ersten Antriebsmotors 1 ist drehfest mit einer Nabe 21 verbunden, welche drehfest mit den drehenden Teilen 20 der Bremse 19 verbunden ist. Eine Druckplatte 22 wird über die Federkraft der Federn 23 auf das drehende Teil 20 gedrückt, wodurch die Bremse im Schließsinne betätigt wird. Durch elektrisches Beaufschlagen des Elektromagneten 24 wird die Druckplatte 22 vom drehenden Teil 20 gelöst, wodurch die Bremse im Öffnungssinne betätigt wird.
Fig. 3:
   Die Antriebswelle 2 des ersten Antriebsmotors 1 ist über eine Paßfeder 25 mit der Nabe 21 verbunden. Die Nabe 21 ist mit dem drehenden Teil 20 der Bremse 19 verbunden. Durch die Federkraft der Tellerfeder 26 wird die Druckplatte 22 auf das drehende Teil 20 gedrückt, wodurch die Bremse im Schließsinne betätigt wird. Durch Druckbeaufschlagung des Kolbens 27 über die Zuführung 28 wird die Druckplatte 22 über einen Stößel 29 von dem drehenden Teil 20 gelüftet, wodurch die Bremse im Öffnungssinne betätigt wird.
Fig 4:
   Die Antriebswelle 2 des ersten Antriebsmotors 1 weist eine Mitnahmeverzahnung 30 auf, über welche drehende Teile 20, die sogenannten Bremslamellen, drehfest verbunden sind. Die Bremse wird, wie die Bremse in Fig. 3, über eine Tellerfeder 26 im Schließsinne und hydraulische Druckbeaufschlagung über die Zuführung 28 im Öffnungssinne betätigt.
Fig. 5:
   Die Bremse nach Fig. 5 entspricht der Bremse nach Fig. 2, wobei das drehende Teil 20 über eine Mitnahmeverzahnung 30 mit der Antriebswelle 2 drehfest verbunden ist.

### Bezugszeichen

- 1: erster Antriebsmotor
- 2: Antriebswelle
- 3: erstes Stirnrad
- 4: Untersetzungsgetriebe
- 5: zweites Stirnrad
- 6: Abtrieb
- 7: Antriebsrad
- 8: zweiter Antriebsmotor
- 9: Antriebswelle
- 10: inneres Zentralrad
- 11: Planetengetriebe
- 12: Planeten
- 13: erstes Hohlrad
- 14: zweites Hohlrad
- 15: Deckel
- 16: Deckel
- 17: Gehäuse
- 18: Lenkgetriebe
- 19: Bremse
- 20: drehende Teile
- 21: Nabe
- 22: Druckplatte
- 23: Federn
- 24: Elektromagnet
- 25: Paßfeder
- 26: Tellerfeder
- 27: Kolben
- 28: Zuführung
- 29: Stößel
- 30: Mitnahmeverzahnung

## Patentansprüche

1. Radantrieb, insbesondere für ein Flurförderfahrzeug, mit einem ersten elektrischen Antriebsmotor (1), welcher über mindestens ein Stirnradgetriebe (4) einen Abtrieb (6) im Sinne eines Fahrantriebs antreibt, welcher mit einem Fahrzeugrad (7) verbunden ist, mit einem zweiten elektrischen Antriebsmotor (8), dessen Abtriebswelle (9) so mit dem Abtrieb (6) in Verbindung steht, dass durch Drehung der Abtriebswelle (9) der Abtrieb (6) im Sinne einer Lenkbewegung gedreht wird, und einer Bremse (19), bei deren Betätigung im Schließsinne der Radantrieb bremsbar ist, **dadurch gekennzeichnet, dass** der erste Antriebsmotor (1), der zweite Antriebsmotor (8) und die Bremse (19) koaxial zur Abtriebswelle (9) des zweiten Antriebsmotors (8) angeordnet sind, und die Bremse (19) zwischen dem ersten Antriebsmotor (1) und dem zweiten Antriebsmotor (8) angeordnet ist.

2. Radantrieb nach Anspruch 1, **dadurch gekennzeichnet, dass** die Abtriebswelle (9) ein inneres Zentralrad (10) eines Planetengetriebes (11) antreibt, dessen Planetenräder (12) mit einem ersten Hohlrad (13) und einem zweiten Hohlrad (14) in Wirkverbindung stehen, welche unterschiedliche Zähnezahlen aufweisen, wobei das erste Hohlrad (13) mit einem Bauteil des Fahrzeugs und das zweite Hohlrad (14) mit einem drehbaren Bauteil (16) in Verbindung stehen.

3. Radantrieb nach Anspruch 2, **dadurch gekennzeichnet, dass** das Planetengetriebe (11) koaxial zum ersten Antriebsmotor (1) angeordnet ist.

4. Radantrieb nach Anspruch 2, **dadurch gekennzeichnet, dass** das drehbare Bauteil (16) mit einem Gehäuse (17) des Abtriebs (6) in Verbindung steht.

5. Radantrieb nach Anspruch 1, **dadurch gekennzeichnet, dass** der erste Antriebsmotor (1), der zweite Antriebsmotor (8) und die Bremse (19) in einem gemeinsamen Gehäuse angeordnet sind.

6. Radantrieb nach Anspruch 1, **dadurch gekennzeichnet, dass** die Bremse (19) über Federkraft im Schließsinne und über elektromagnetische Kraft oder hydraulische Kraft im Öffnungssinne betätigbar ist.

7. Radantrieb nach Anspruch 6, **dadurch gekennzeichnet, dass** die Federkraft von mindestens einer Tellerfeder (26) oder mindestens einer Schraubendruckfeder (23) erzeugt wird.

8. Radantrieb nach Anspruch 1, **dadurch gekennzeichnet, dass** die Bremse (19) als eine flüssigkeitsgekühlte Scheibenbremse ausgeführt ist.

9. Radantrieb nach Anspruch 1, **dadurch gekennzeichnet, dass** die Bremse (19) als eine trockenlaufende Scheibenbremse ausgeführt ist, wobei Dichtmittel zwischen der Bremse (19) und einem Untersetzungsgetriebe (4) angeordnet sind.

10. Radantrieb nach Anspruch 1, **dadurch gekennzeichnet, dass** der erste Antriebsmotor (1) eine Antriebswelle (2) aufweist, welche über Verbindungsmittel, wie eine Mitnahmeverzahnung oder eine Paßfeder, mit der Bremse in Verbindung steht.

## Claims

1. Wheel drive, in particular for an industrial conveying vehicle, having a first electric drive motor (1) which, via at least one spur gearing (4), drives an output (6) in the sense of a travel drive, which is connected to a vehicle wheel (7), and having a second electric drive motor (8), whose drive shaft (9) is so connected to the output (6) that, through rotation of the output shaft (9), the output (6) is rotated in the sense of a steering movement, and having a brake (19), upon whose actuating in the closing direction the wheel drive can be braked, **characterized in that** the first drive motor (1), the second drive motor (8) and the brake (19) are disposed coaxially relative to the output shaft (9) of the second drive motor (8), and the brake (19) is disposed between the first drive motor (1) and the second drive motor (8).

2. Wheel drive according to Claim 1, **characterized in that** the output shaft (9) drives an inner sun gear (10) of a planetary gearing (11) whose planet gears (12) are operatively connected to a first annulus gear (13) and to a second annulus gear (14) which have different numbers of teeth, the first annulus gear (13) being connected to a component of the vehicle and the second annulus gear (14) being connected to a rotatable component (16).

3. Wheel drive according to Claim 2, **characterized in that** the planetary gearing (11) is disposed coaxially relative to the first drive motor (1).

4. Wheel drive according to Claim 2, **characterized in that** the rotatable component (16) is connected to a housing (17) of the output (6).

5. Wheel drive according to Claim 1, **characterized in that** the first drive motor (1), the second drive motor (8) and the brake (19) are disposed in a common housing.

6. Wheel drive according to Claim 1, **characterized in that** the brake (19) can be actuated in the closing direction by spring force, and can be actuated in the opening direction by electromagnetic force or hydraulic force.

7. Wheel drive according to Claim 6, **characterized in that** the spring force is generated by at least one disc spring (26) or at least one helical compression spring (23).

8. Wheel drive according to Claim 1, **characterized in that** the brake (19) is realized as a liquid-cooled disc brake.

9. Wheel drive according to Claim 1, **characterized in that** the brake (19) is realized as a dry-running disc brake, sealing means being disposed between the brake (19) and a reduction gearing (4).

10. Wheel drive according to Claim 1, **characterized in that** the first drive motor (1) has a drive shaft (2), which is connected to the brake via connecting means, such as a driver toothing or a parallel key.

## Revendications

1. Entraînement de roue, en particulier pour un véhicule de manutention, comprenant un premier moteur d'entraînement électrique (1), lequel entraîne, par l'intermédiaire d'au moins un train de roues droites (4) un organe de sortie (6) constituant un entraînement de roulement qui est relié à une roue (7) du véhicule, un second moteur d'entraînement électrique (8) dont l'arbre de sortie (9) est relié à l'organe de sortie (6) de telle manière que, sous l'effet de la rotation de l'arbre de sortie (9), l'organe de sortie (6) soit mis en rotation pour donner un mouvement de direction, et un frein (19) lors de l'actionnement duquel, dans son sens de fermeture, l'entraînement de roue peut être freiné, **caractérisé en ce que** le premier moteur d'entraînement (1), le second moteur d'entraînement (8) et le frein (19) sont disposés coaxialement à l'arbre de sortie (9) du second moteur d'entraînement (8) et le frein (19) est disposé entre le premier moteur d'entraînement (1) et le second moteur d'entraînement (8).

2. Entraînement de roue selon la revendication 1, **caractérisé en ce que** l'arbre de sortie (9) entraîne une roue centrale intérieure (10) d'un train épicycloïdal (11) dont les roues satellites (12) sont en liaison active avec une première couronne à denture intérieure (13) et une seconde couronne à denture intérieure (14) qui présentent des nombres de dents différents, la première couronne à denture intérieure (13) étant en liaison avec un composant du véhicule et la seconde couronne à denture intérieure (14) avec un composant rotatif (16).

3. Entraînement de roue selon la revendication 2, **caractérisé en ce que** le train épicycloïdal (11) est disposé coaxialement au premier moteur d'entraînement (1).

4. Entraînement de roue selon la revendication 2, **caractérisé en ce que** le composant rotatif (16) est en liaison avec un carter (17) de l'organe de sortie (6).

5. Entraînement de roue selon la revendication 1, **caractérisé en ce que** le premier moteur d'entraînement (1), le second moteur d'entraînement (8) et le frein (19) sont disposés dans un carter commun.

6. Entraînement de roue selon la revendication 1, **caractérisé en ce que** le frein (19) peut être actionné dans le sens de la fermeture au moyen d'une force élastique et dans le sens de l'ouverture au moyen d'une force électromagnétique ou d'une force hydraulique.

7. Entraînement de roue selon la revendication 6, **caractérisé en ce que** la force élastique est produite par au moins une rondelle Belleville (26) ou par au moins un ressort de compression hélicoïdal (23).

8. Entraînement de roue selon la revendication 1, **caractérisé en ce que** le frein (19) est constitué par un frein à disque refroidi par liquide.

9. Entraînement de roue selon la revendication 1, **caractérisé en ce que** le frein (19) est constitué par un frein à disque fonctionnant à sec, des moyens d'étanchéité étant disposés entre le frein (19) et un mécanisme réducteur (4).

10. Entraînement de roue selon la revendication 1, **caractérisé en ce que** le premier moteur d'entraînement (1) comporte un arbre d'entraînement (2) qui est en liaison avec le frein par l'intermédiaire de moyens de liaison tels qu'une denture d'entraînement ou une clavette.
